# EUROPEAN PATENT APPLICATION

(11) **EP 1 568 386 A1**
(43) Date of publication of application: **31.08.2005**
(21) Application number: 03772818.5
(22) Date of filing: 17.11.2003
(51) Int. Cl.: A61L 27/36

(54) **METHOD OF ORGAN REGENERATION**

(30) Priority: 15.11.2002 JP 2002332530
(71) Applicant: Kyushu TLO Company, Limited, Fukuoka 812-0053 (JP); Ishikawa, Fumihiko, Fukuoka 810-0022 (JP)
(72) Inventor: ISHIKAWA, Fumihiko, Fukuoka-shi, Fukuoka 810-0022 (JP); HARADA, Mine, Fukuoka-shi, Fukuoka 814-0001 (JP)
(74) Representative: Woods, Geoffrey Corlett
(86) International application number: PCT/JP2003/014581
(87) International publication number: WO 2004/045666

(57) **Abstract**

It is intended to provide a method of regenerating an organ or a part thereof which comprises transplanting bone marrow or hematopoietic stem cells into an injured mammal or a mammal having an injured in organ or a part thereof; a method of treating injury: a method of producing an organ or a part thereof; and an organ or a part thereof produced by this method.

## Description

### TECHNICAL FIELD

The present invention relates to a method of organ regeneration.

### BACKGROUND ART

Regenerative medicine is a therapeutic method of curing organs or tissues lost in diseases or accidents utilizing artificially cultured cells or the like. This therapeutic method has less side effect than existing medicines, and yet is expected to be applicable to treatment of incurable diseases such as Alzheimer's disease. It is said that regeneration of skin is the most promising regeneration to be put into practice. There has been reported a success in generating the structure of derm by separating fibroblast cells from derm, mass-culturing those cells and seeding the resultant cells on a collagen sheet. If such a sheet is transplanted in a patient with injury in the skin (such as bum injury), it is believed that the sheet will be integrated into the patient's skin to thereby regenerate that skin.

Regeneration medicine using stem cells has also been tried. Stem cells are undifferentiated cells with both self-proliferation capacity and differentiation capacity. They are sources from which tissues or organs develop, and are present in almost all organs or tissues. Among various stem cells such as hematopoietic or neural stem cells, ES cells (embryonic stem cells) have high proliferative capacity and are capable of differentiating into almost all types of tissues. ES cells are prepared from early embryos (fertilized eggs) about 5 to 7 days after fertilization in the case of human and about 3 to 4 days after fertilization in the case of mouse. Since ES cells have capacity to differentiate into various cells and high proliferation capacity, their application to a new therapeutic method of regenerating lost cells (regenerative medicine) is expected. However, ES cells have two major problems. One is an ethical problem that they are obtained from fertilized eggs. The other is that, at present stage, it is extremely difficult to control ES cells so that they differentiate into only necessary cells. Under circumstances, the present inventors have chosen bone marrow-derived stem cells to further investigate into regenerative medicine.

The liver is the only organ capable of wide-ranged regeneration. Until recently, it was believed that the regeneration of the liver is only performed by hepatocytes or egg cells. It is known that oval cells (cholangiole cells) present in the Herring duct intercalated between bile capillaries in the liver tissue has bipotency to differentiate into hepatocytes and bile duct cells. Further, Petersen et al. revealed that it is highly possible that bone marrow-derived cells generate hepatocytes after administration of carbon tetrachloride and allyl alcohol. Lagasse et al. show that bone marrow transplantation into a tyrosinemia model mouse causes differentiation of its hepatocytes, resulting in partial improvement of the liver function (see Lagasse E. et al., Nat. Med. 2000 6(11): 1229-1234).

However, it has not been proved that stem cells having the characteristic of oval cells were used in the regeneration of diseased or damaged liver tissue. Besides, the following points have not yet been elucidated: can oval cells really be stem cells in the liver tissue and play a major role in the regeneration of the liver tissue; or to what extent hematopoietic stem cells can re-constitute diseased liver tissue and what situation do they need to differentiate into hepatocytes or other cells constituting the liver?

### DISCLOSURE OF THE INVENTION

The present invention aims at providing a method of regenerating organs, a method of treating organs and a method of preparing organs, as well as regenerated organs.

As a result of intensive and extensive researches toward the solution of above problems, the present inventors have found that bone marrow transplantation into a mammal with an impairment makes it possible for the organ with the impairment to regenerate at a high ratio. Thus, the present invention has been achieved.

The present invention relates to the following:
(1) A method of regenerating an organ or a part thereof, comprising performing bone marrow transplantation or hematopoietic stem cell transplantation in a mammal having an impairment in the organ or the part thereof.
(2) A method of treating an impairment, comprising performing bone marrow transplantation or hematopoietic stem cell transplantation in a mammal having the impairment in an organ or a part thereof and thereby regenerating the organ or the part thereof.
(3) A method of preparing an organ or a part thereof, comprising performing bone marrow transplantation or hematopoietic stem cell transplantation in a mammal having an impairment in the organ or the part thereof, thereby regenerating the organ or the part thereof, and recovering the resultant regenerated organ or the part thereof.
(4) An organ or a part thereof which has been regenerated by performing bone marrow transplantation or hematopoietic stem cell transplantation in a mammal having an impairment in the organ or the part thereof.

In the methods described in (1) to (3) above and the organ or the part thereof described in (4) above, cells used in the bone marrow transplantation may be bone marrow cells, and hematopoietic stem cells used in the hematopoietic stem cell transplantation may be derived from, for example, peripheral blood or cord blood (e.g. peripheral blood stem cells or cord blood stem cells). With respect to the impairment, functional disorders or physical or chemical injuries may be enumerated, for example. The mammal is not particularly limited. For example, a new born mammal may be used. The organ is at least one selected from the group consisting of liver, heart, brain, lung, kidney, intestine, pancreas, eye, bone and tooth.

### BRIEF DESCRIPTION OF THE DRAWINGS

Fig. 1 provides photographs showing regeneration of the liver.
Fig. 2 provides photographs showing regeneration of the liver at the resected stump.
Fig. 3 provides photographs showing differentiation of a large number of bone marrow-derived cells and appearance of individual myocardial cells in the endocardium.
Fig. 4 is a photograph showing the presence of about 4 to 6 myocardial cells in a piece of coronary cross section.
Fig. 5 is a photograph created by analyzing coronary cross sections for the presence of individual myocardial cells and preparing three-dimensional (3D) images. This photograph shows stereoscopically that a great number of bone marrow stem cell-derived myocardial cells are distributed throughout the heart.
Fig. 6 provides photographs showing the results of staining of myocardial cells with connexin 43 and troponin 1c, and photographs showing the results of observation of GFP positive myocytes by Nomarski imaging.
Fig. 7 is a photograph showing regeneration of lung and bronchus epithelial cells in a bone marrow-derived manner.
Fig. 8 provides photographs showing regeneration of mesangial cells in the kidney.
Fig. 9 provides photographs showing regeneration of cells in the small intestine.
Fig. 10 provides photographs showing regeneration of osteocytes and osteoblast cells in the bone.
Fig. 11 provides photographs showing regeneration of the gingiva and the tooth.
Fig. 12 provides photographs showing regeneration of the surface layer of the eye.
Fig. 13 is a photograph showing regeneration of the ectocomea of the eye.
Fig. 14 is a photograph showing regeneration of nerve cells in the brain.

### BEST MODE FOR CARRYING OUT THE INVENTION

Hereinbelow, the present invention will be described in detail.

The present invention demonstrates that, by performing bone marrow transplantation or hematopoietic stem cell transplantation (e.g. peripheral blood hematopoietic stem cell transplantation) in a mammal having an impairment in an organ or a part thereof, hematopoietic stem cells are differentiated into those cells constituting the organ at a high ratio and in a wide range, resulting in the regeneration of the organ.

Hitherto, it was believed that no division or regeneration occurs in the heart or the brain after birth. The present invention has succeeded for the first time in preparing (regenerating) an organ by creating a mammal injury model and performing hematopoietic stem cell transplantation therein.

Further, once the organs of new born mice have matured, stem cells in individual tissues (such as liver, heart, etc.) exhibit their functions fully. Therefore, it is believed that in the regeneration of an injured tissue, stem cells existing in that tissue play a major role in the regeneration rather than hematopoietic stem cells. The present inventors have paid attention to the immature environment surrounding newborns. In the immediately after birth when organs in the body grow rapidly, proliferation of individual cells constituting tissues is remarkable. In view of that hematopoietic stem cells transplanted in newborns will be utilized in tissues at a high probability and that newborns are very likely to have immature environment with high plasticity, the present inventors performed hematopoietic stem cell transplantation in newborns and also created injury models to examine regeneration.

In order to put into practice the regenerative medicine using the pluripotency of hematopoietic stem cells, the present inventors considered regeneration of the liver at first. For the purpose of preparing highly pure regenerated livers, the livers of newborns (recipients) were partially excised and then bone marrow transplantation (in particular, transplantation of stem cells in the bone marrow) was performed. Analysis of the livers of these recipients revealed that a great number of hepatocytes have been differentiated from the donor-derived hematopoietic stem cells. As a result, regeneration of the liver using hematopoietic stem cells has become possible. Thus, it has been found that the differentiation capacity of hematopoietic stem cells can be manifested even in patients with impairments. The present invention has been achieved based on such finding, and will lead to development of regenerative medicine using hematopoietic stem cells in various organs.

### 1. Mammals

Specific examples of mammals which may be used in the present invention include pig, bovine, horse, monkey, dog, sheep, goat, rat and mouse. Among all, mouse is preferable because model animals are abundant and inbred lines are established. Pig is also preferable because it is suitable for application to practical regenerative medicine. Clinical application to human is also possible. In this case, first, informed consent must be obtained and then patients should be selected under strict control of doctors. Newborn mammals used in the present invention are not particularly limited. Newborns preferably within 4 days after birth, more preferably within 2 days after birth, may be used.

### 2. Organs or Parts Thereof

In the present invention, the term "organ" refers to every tissue or organ necessary for performing life activities in the body and includes parts (e.g. cells, tissues, etc.) of organs.

As organs, various tissues and organs in the digestive system, respiratory system, urinary system, genital organs, cardiovascular system, lymph system, sense organ system, central nerve system, skeletal system, and muscles may be enumerated, for example.

Specific examples of above organs include, but are not limited to, the following.

Digestive system: oral cavity, throat, esophagus, stomach, small intestine, large intestine, liver, pancreas
Respiratory system: trachea, bronchus, lung, pleura
Urinary system: kidney, ureter, urinary bladder
Genital organs: internal sex organs, external sex organs
Cardiovascular system: heart, artery, vein
Lymph system: lymph vessel, lymph node, spleen, thymus
Sense organ system: visual organs (eye: oculus, accessory ocular organs, etc.), hearing organs (eardrum)
Central nerve system: brain (cerebrum, diencephalon, mesencephalon, cerebellum), medulla, spinal cord
Skeletal system: skull, backbone, rib, sternum, bone of upper limb, humerus, bone of lower limb, femur, etc.
Muscles: skeletal muscle, smooth muscle, etc.
Others: skin, tooth, gingiva

### 3. Impairments in Organs

In the present invention, the term "impairment" refers to a functional disorder or a physical or chemical injury occurring in one of the above-mentioned organs or a part thereof. The term "a part" used herein means a certain amount of the relevant organ which can be injured or resected without making it impossible for the organ to maintain its function. This amount varies depending on the organ. In the case of the liver, for example, 0.1-50%, preferably 10-30% of the total organ is the "part" that may be excised in the present invention. According to an international classification of impairments, "functional disorder" means a problem in psychosomatic function or bodily structure, such as a remarkable variation or loss. This term encompasses a disease state in which abnormality has occurred in the above-mentioned organs or a part thereof. The term "physical or chemical injury" means a physical or chemical damage to an organ or a part thereof; such damage includes those which are caused by excising with a surgical knife, pricking with a needle, picking and peeling off with tweezers, exposing to high concentration oxygen, laser irradiation, and irradiation. Wounds which occur when tissue samples for biopsy have been taken are also included in the "injury".

Specific examples of injury include excision of hepatic lobes with a surgical knife in the liver; wall-penetrating injury caused by puncture into the cardiac cavity in the heart; injury caused by direct pricking with a needle around the ventricle in the brain; and injury to the epithelium in the lung caused by exposure to high concentration oxygen. Injuries may be given to these organs by conventional methods such as surgical operation, or operation using an endoscope or celoscope.

### 4. Bone Marrow Transplantation or Hematopoietic Stem Cell Transplantation

Cell transplantations performed in the present invention are classified into bone marrow transplantation, peripheral blood stem cell transplantation and cord blood stem cell transplantation, depending on the cell transplanted. Therefore, cells which may be used in the present invention are bone marrow cells, peripheral blood stem cells and cord blood stem cells.

Bone marrow is a tissue present in the medullary cavity formed by osteoclast cells located inside of the bone tissue, and is a major hematopoietic tissue. In bone marrow, progenitor cells for the entire blood cell lineage cells (erythrocytes, granulocytes, monocytes-macrophages, megakaryocytes-platelets, mast cells, lymphocytes) are present, and released into the peripheral blood when they have matured and differentiated. These progenitor cells are derived from hematopoietic stem cell. Peripheral blood stem cells are released into the blood after chemotherapy (administration of anti-cancer drugs), or after the administration of granulocyte-stimulating factor that induces increase in leukocytes. Cord blood stem cells are hematopoietic stem cells present in the blood in the umbilical cord.

When bone marrow transplantation is selected in the present invention, mammal-derived bone marrow cells useful in the transplantation include bone marrow cells from any mammal including human. In this case, the donor (supplier of the bone marrow) may be either allogeneic or heterogeneic to the recipient (receiver of the bone marrow).

Bone marrow cells may be collected by conventional methods such as bone marrow aspiration. The resultant bone marrow cells may be used as they are, or suspension cells alone may be used. When suspension cells are used, bone marrow cells are suspended in a culture broth (animal cell culture medium preferably containing 10% fetal calf serum), seeded on plastic dishes and cultured. By these operations, adhesive cells are adhered to the dishes, making it possible to recover only suspension cells. The thus obtained culture broth containing suspension cells is centrifuged to recover only suspension cells. As an animal cell culture medium, DMEM, RPMI-1640, HamF12 broth or a mixture thereof may be used. In the present invention, it is also possible to transplant only hematopoietic stem cells or mesenchymal stem cells from bone marrow. These hematopoietic stem cells or mesenchymal stem cells are also included in the "bone marrow cells" in the bone marrow transplantation of the invention.

If transplantation of peripheral blood stem cells or cord blood stem cells is selected in the present invention, peripheral blood stem cells and cord blood stem cells may be collected by known methods. As mammal-derived stem cells to be used in the peripheral blood stem cell or cord blood stem cell transplantation, stem cells of any mammal including human may be used. Like in the case of bone marrow transplantation, the donor may be either allogeneic or heterogeneic to the recipient.

In the present invention, it is also possible to select CD34⁺ cells, CD34⁺CD38⁻ cells, side population (SP) cells, monocytes or the like from bone marrow cells (including hematopoietic stem cells and mesenchymal stem cells in bone marrow), peripheral blood stem cells and cord blood stem cells, if desired or necessary.

For the separation of cells for use in the transplantation (hematopoietic stem cells) from bone marrow, peripheral blood or cord blood, cells may be labeled with known surface antigens and subjected to sorting by flowcytometry to thereby isolate necessary hematopoietic stem cells alone at a purity of 95% or more. For the separation of mesenchymal stem cells from bone marrow cells, adhesive cells may be separated in the above-described culture. Alternatively, separation methods using antibodies are also possible. It is known that mesenchymal stem cells are close to ES cells in their capacity and that they differentiate into cells of bone, cartilage, fat, heart, nerve, liver, and so on. Thus, they are attracting attention as the "second omnipotent cell". Peripheral blood stem cells may be obtained by apheresis.

The time period from a partial injury of an organ to transplantation may be within the range from 0 to 1 week, preferably 0 to 96 hrs (4 days), more preferably 0 to 48 hrs (2 days).

The bone marrow cells or hematopoietic stem cells prepared as described above are transplanted in a mammal with an impairment or a recipient (mammal) in which a part of an organ has been injured in advance. The method of transplantation is as described below.

First, pretreatment is performed. Administration of a high dose of anti-cancer drug or total body irradiation is carried out sometime in the period from 48 hrs to up to immediately before the transplantation, to thereby almost completely destroy the bone marrow cells, etc. in the recipient. On the day of transplantation, the bone marrow fluid, peripheral blood stem cells, cord blood stem cells or the like supplied by the donor is drip-fed into the vein of the recipient.

The recipient is kept under control in a clean room until normal blood components have been generated and its conditions have been stabilized. After the transplantation, the recipient may die early because of rejection, GVH disease (graft-versus-host disease), severe infection, etc. Therefore, the progress is observed continuously and, if necessary, immunosuppressant, antibiotics, or the like is administered.

### 5. Regeneration of Organs

Organs are allowed to regenerate after the bone marrow transplantation or hematopoietic stem cell transplantation performed as described above. The term "regeneration" means that new cells, tissues or organs which are composed of donor-derived cells are newly born from the injured site. Since regeneration periods vary depending on the organ, the presence or absence of regeneration is appropriately observed and organs showing regeneration are allowed to regenerate up to pre-determined sizes. For example, if 30% of the liver has been resected the liver is allowed to regenerate for 48 hrs to several weeks. In this case, it is believed that transplanted stem cells are accumulated at the injured site because of the resection and continue differentiation and proliferation even after the liver has been regenerated. In the case of the heart, it is impossible to perform resection as performed in the liver. However, puncture into the cardiac cavity in newborns is believed to be a sufficient injury. The period necessary for the regeneration is from several hours to 2 weeks. In the regenerative process of the heart, it is preferable to examine whether or not stem cells have a definite morphology of cardiac muscle or to examine the time period required for obtaining the morphology of cardiac muscle.

For testing whether or not an organ is derived from a donor, any of the known techniques may be used. For example, donor's bone marrow cells may be labeled with GFP (green fluorescence protein) or a similar material and then whether the label is expressed in the relevant organ of the recipient may be observed. The judgment on whether or not individual organs have been regenerated in a donor-derived manner may made by immunostaining. This is a direct and sure method of discrimination though the size of cells can be an indicator.

When the relevant organ has grown (regenerated) to a pre-determined size, the organ is allowed to take in the recipient if it is clinical application. Alternatively, when an experimental animal was used, the organ is removed from the animal by surgical operation or the like. In this case, it is not necessary to wait until the organ has regenerated to a pre-determined size. A part of the tissue or cells may be recovered in the course of regeneration, if necessary.

In the present invention, when the liver is regenerated, it is possible to obtain from bone marrow-derived stem cells not only hepatocytes but also hepatic stellate cells, Kupffer cells, endothelial cells, and bile duct cells. Besides, the regenerated part of the liver has donor-derived cells and highly pure. The organ removed as described above is used in organ transplantation, etc. in the donor (supplier of bone marrow in the case of bone marrow transplantation).

Hereinbelow, the present invention will be described in more detail with reference to the following Examples. However, the technical scope of the present invention is not limited to these Examples.

### EXAMPLE 1: Regeneration of Liver

### (1) Mice

GFP (green fluorescent protein)-transgenic mice (hereinafter, called "GFP mice") were prepared by ligating a DNA encoding GFP to Actin promoter (Okabe M. et al., FEBS Lett. 1997; 407(3):313-319). These GFP mice were used as donors in bone marrow transplantation. GFP is expressed in every type of cells in the body. Therefore, when cells derived from these mice have been transplanted in recipients, it is possible to identify donor-derived cells by detecting GFP positive cells.

C57/BL6 mice were purchased from Charles River Japan and used as recipients in bone marrow transplantation.

Both mice were bred in animal facilities of Kyushu University under specific control.

### (2) Preparation of Bone Marrow Cells

Bone marrow cells to be transplanted were prepared from GFP mice. Briefly, after dissection of GFP mice, bone marrow cells were collected from thighs and shinbones. The collected donor cells were passed through a 25-gauge needle and a 40 µm mesh filter repeatedly to thereby prepare a single cell suspension. In order to isolate hematopoietic progenitor cells, cells were cultured at 4°C for 30 min with antibodies such as B220, CD3, Gr-1, Mac-1 and TER119. Then, after washing with 2% fetal calf serum (FCS)-containing PBS, bone marrow cells were cultured with sheep-anti-rat immuno-magnetic beads (sheep-anti-rat IgG conjugated Dynabeads; M-450 DYNAL Great Neck, NY). Cells not binding to the beads were collected for further separation of Sca-1(+) cells. Since Sca-1 is one of the most important markers for mouse hematopoietic stem cells, Lin(-)Sca-1(+) cells were separated from the total bone marrow cells of GFP mice. Positive selection of Sca-1(+) cells was performed on rat-anti-mouse Sca-1 antibody-conjugated microbeads. The resultant Lin(-)Sca-1(+) cells were suspended in 50 µl of PBS.

### (3) Partial Liver Excision

Newborn mice (C57/BL6) within 24 hrs after birth were anesthetized by intraperitoneal injection of 200 µg of ketamine chloride. After making a 1 cm incision in the skin, almost one half of the hepatic lobes (about 10-25% of the whole liver) was removed. The skin and the peritoneum were sutured with nylon thread.

### (4) Transplantation of Hematopoietic Stem Cells

Newborn mice (C57/BL6) were treated with 500 Gy total body irradiation. Within 6 hrs after the irradiation to the recipient newborn mice (within 24 hrs after the liver excision), GFP mice-derived Lin(-) Sca-1(+) cells prepared as described above (5000 cells) were transplanted into each of the newborn C57/BL6 mice through the facial vein.

### (5) Experiment on Chimera Phenomenon of Hematopoietic Stem Cells

Two to eight months after the transplantation, peripheral blood was collected from the retro-orbital venous plexus, and bone marrow cells were collected from the lower limbs of recipient mice.

Donor mice-derived cells were detected as GFP positive cells using FACS Calibur (Becton Dickinson). For the lineage expression of donor-derived cells, peripheral blood or bone marrow cells were stained with B220, CD3, Gr-1, Mac-1 and TER119.

### (6) Analysis of Differentiation in Liver

Mice 3 to 60 days after the bone marrow transplantation were anesthetized by isoflurane inhalation and euthanized by cervical dislocation. Immediately after dissection, the liver was fixed in 4% paraformaldehyde (PFA) at room temperature for 30 min. The fixed tissue was dehydrated with graded alcohol series and then sliced into sections 50 µm thick using a vibratome. Also, the total tissue was fixed in 4% paraformaldehyde (PFA) at room temperature for 10 min and frozen in OCT (optical cutting temperature) compound (10.24% polyvinyl alcohol, 4.26% polyethylene glycol, 85.5% non-reactive ingredients). This sample was used for preparing thin sections of 4-6 µm.

### (7) Immunofluorescence

Tissue sections were treated as described below depending on the thickness. Sections 50 µm thick were stained with an antibody and incubated with a primary antibody overnight at 4°C. After washing with PBS twice in 2 hrs, the sections were reacted with a secondary antibody bound to Cy-3 (Jackson Immunoresearch).

Sections 4-6 µm thick were stained with an antibody and reacted with a primary antibody for 1 hr at room temperature. After washing, the tissue sections were incubated with a secondary antibody bound to Cy-3.

Immunostaining was analyzed carefully under a confocal microscope (Olympus).

### (8) Results

### (i) Analysis of the Chimera Phenomenon

Lin(-) Sca-1(+) cells were transplanted in each of the newborn recipient mice, and the resultant chimera phenomenon in hematopoietic cells in the recipient mice was analyzed. Every recipient mouse exhibited a chimera phenomenon of donor cell type in 70% or more of its hematopoietic cells.

Further, donor-derived cells were separated from recipient bone marrow cells and transplanted in secondary newborn recipient. This means that bone marrow cells include hematopoietic stem cells having self-regeneration capacity.

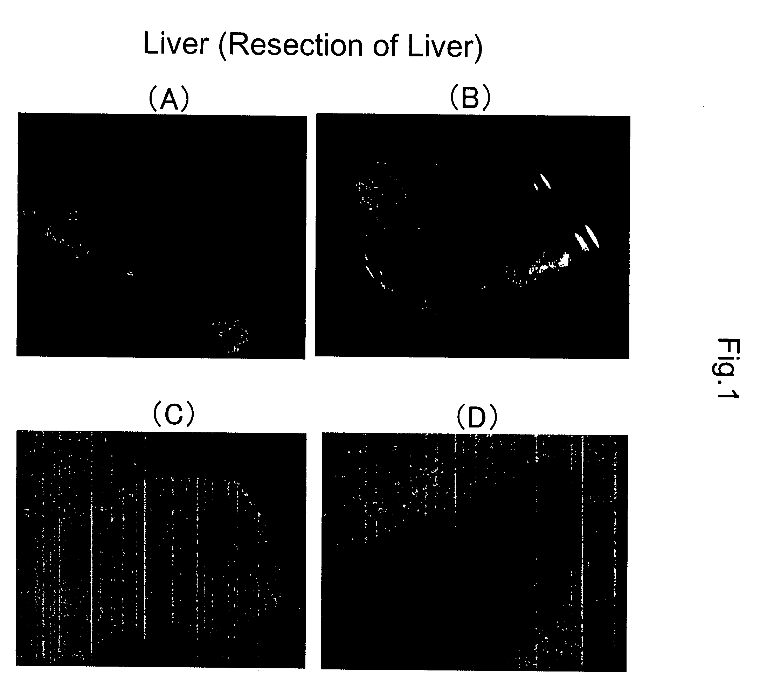

In order to identify the distribution and external appearance of GFP positive cells, the liver was observed under a fluorescent microscope at high magnifications and low magnifications immediately after dissection (Fig. 1). As a result, bone marrow stem cell-derived GFP positive cells were distributed throughout the liver. This shows that the liver regenerated from the resected stump is derived from bone marrow stem cells at a high ratio. Although most of the GFP positive cells were spindle-shaped, several percent of donor cells appeared to be hepatocytes morphologically. A large number of GFP positive, spindle-shaped cells were distributed around the central vein (Fig. 1).

In Fig. 1A, the object seen at the lower right corner of the field and emitting fluorescence is a regenerated hepatic lobe of the liver. Compared to other hepatic lobes, GFP is strongly positive in this lobe. Fig. 1B shows the external appearance of a regenerated liver. The regenerated liver has external appearance similar to that of normal liver. Fig. 1C and 1D show findings obtained by observing the regenerated liver highly enlarged.

### (ii) Regeneration of Liver

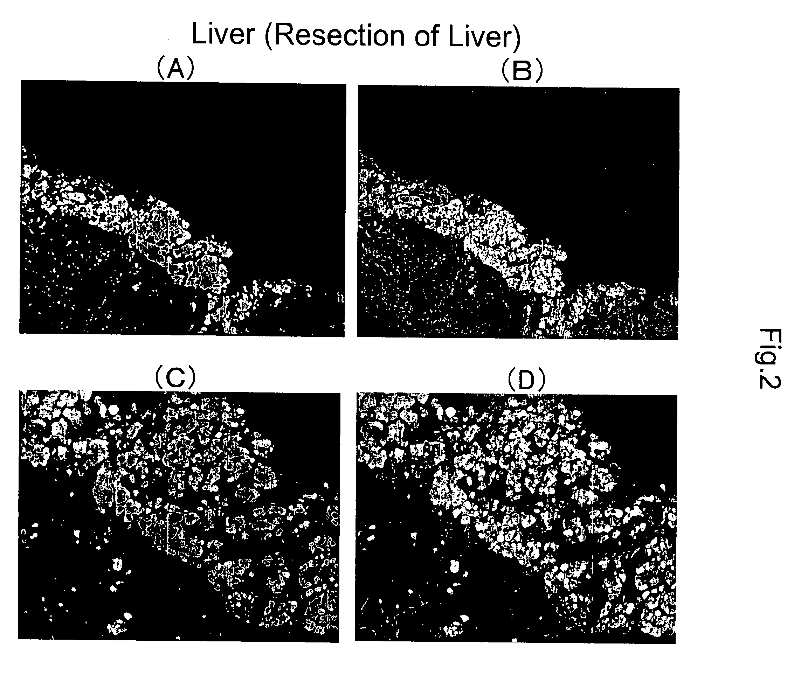

The regenerated hepatic lobe exhibited intense GFP fluorescence at the resected stump (Fig. 2A, 2C). In Fig. 2, panels A and C show the gathering of GFP positive cells at the resected stump of the liver, and panels B and D show the staining of hepatocytes with albumin antibody. Panel C is a highly enlarged image of panel A, and panel D is a highly enlarged image of panel B. As shown in Fig. 2, the regenerated lobe is a donor-derived normal hepatic lobe, and donor-derived cells were present there at a high purity. The results shown in panels B and D proved that albumin positive cells are present at a high ratio because cells present yellow color and that they are functionally normal hepatocytes producing albumin.

### EXAMPLE 2: Regeneration of Heart

### (1) Mice and Bone Marrow Cells

Breeding/administration of mice and preparation of bone marrow cells were carried out in the same manner as in Example 1.

### (2) Partial Injury in Heart

Recipient mice (C57/BL6) immediately to up to 3 days after birth were pricked with a 29-gauge needle to injure the cardiac muscle without opening the chest. The success of this manual technique could be easily conformed by reverse flow of the blood in the cardiac cavity.

### (3) Transplantation of Bone Marrow

Pretreatment (irradiation) of mice and transplantation of hematopoietic cells therein were carried out in the same manner as in Example 1.

### (4) Analysis of Differentiation in Heart

### (i) Preparation of Heart Tissue Samples

Recipient mice were anesthetized by isoflurane inhalation and then euthanized by cervical dislocation. Immediately after dissection, the heart tissue was fixed in 4% paraformaldehyde (at room temperature for 30 min). The fixed tissue was dehydrated with graded alcohol series and then sliced into sections 50 µm thick using a vibratome (Microslicer DTK-1000; DSK). Also, the total tissue was fixed in 4% paraformaldehyde (PFA) (at room temperature for 10 min) and frozen in OCT compound for preparing thin sections of 4-6 µm. After sufficient freezing, the tissue was sliced into sections 6 µm thick using Cryostat (model CM3050S; Leica).

### (ii) Immunostaining

Heart sections were stained with the antibodies described below. In order to identify myocardial cells, heart sections were stained with troponin 1C (connexin 43), sarcomeric actin, connexin 43 or Nkx2.5. When thick sections were stained with an antibody, individual sections were incubated with a primary antibody at 4°C overnight. Then, after washing twice with PBS in 2 hrs, the sections were reacted with a secondary antibody bound to Cy-3 (Jackson Immunoresearch).

When thin sections were stained with an antibody, individual sections were incubated with a primary antibody at room temperature for 1 hr. Then, after washing, the tissue sections were incubated with a secondary antibody bound to Cy-3.

Immunostaining was analyzed under a confocal microscope (Olympus).

### (5) Results

Lin(-) bone marrow cells (5 x 10⁵ cells) were transplanted in each of the newborn recipient mice, and the chimerism in the recipient hematopoietic cells was analyzed 2 or 5 months after the transplantation. As a result, donor cell-type chimerism was observed in every recipient mouse in 70% or more of its hematopoietic cells. Further, donor-derived cells were separated from recipient bone marrow cells and transplanted in secondary recipient newborn mice. Donor-derived cells were also observed in the secondary recipient mice. From these results, it can be said that the bone marrow cells took in the primary recipient mice include hematopoietic stem cells having self-regeneration capacity.

Further, 2 months after the cardiopuncture and the hematopoietic stem cell transplantation, chimerism and changes in differentiation in the heart tissue were analyzed.

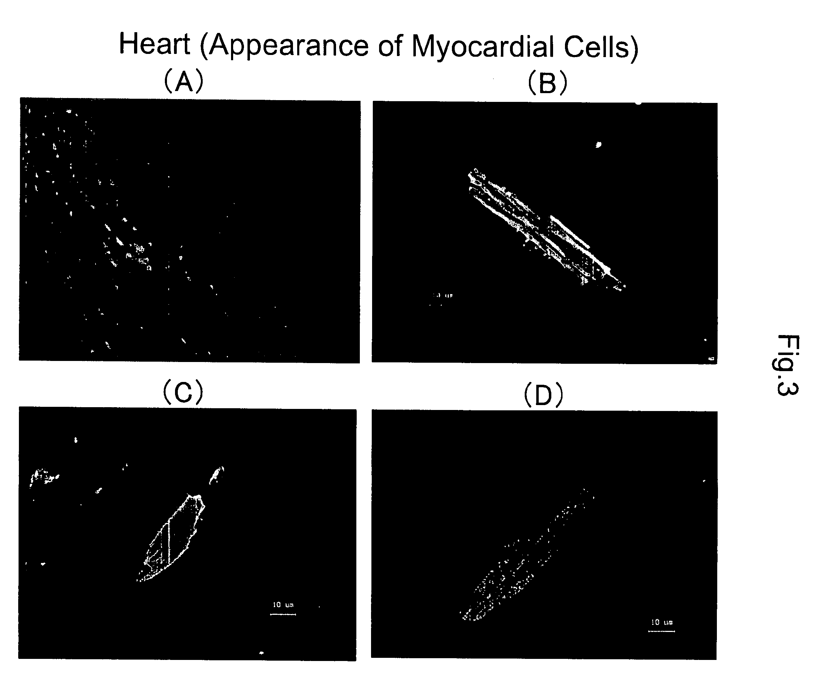

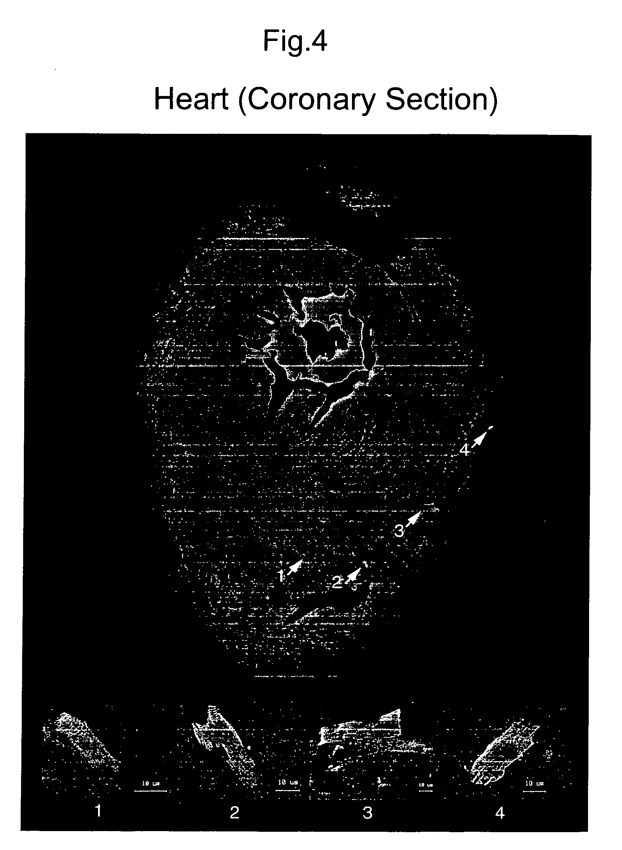

First, the total tissue was observed under a fluorescence microscope at an excitation wave length of 488 nm. In the pericardium, GFP positive cells were identified along the coronary artery. When tissue samples were cut sagittally, GFP positive regions were found along myocardial fibers (Fig. 3). A large number of GFP positive cells were found in a spindle shape (Fig. 3). In Fig. 3, panel A is an image obtained when the endocardium was observed under a fluorescence stereoscopic microscope. A large number of GFP positive cells (bone marrow-derived cells) are recognized. Panels B, C and D show those myocardial cells which definitely have striations among GFP positive cells. When fluorescence observation was conducted again, similar results were obtained. Thus, it was found that the results concerning GFP positive cells have reproducibility and that a large number of myocytes can be used as a heart injury model. Further, as shown in Fig. 4, a plurality of donor-derived myocardial cells were observed in one cross section (arrow marks 1 to 4). When each of them was enlarged, they exhibited a definite morphology of striated muscle (Fig. 4).

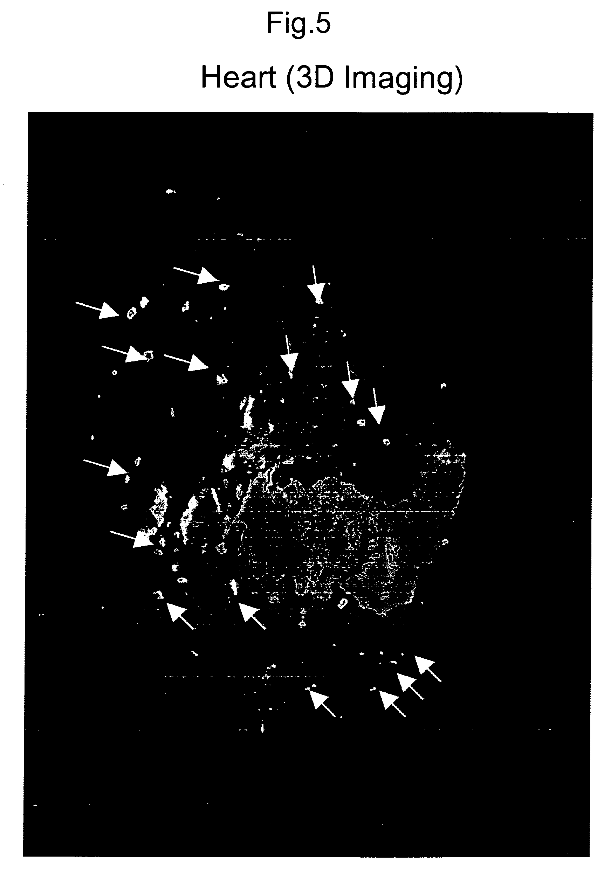

Further, as a result of immunofluorescence analysis of myocardial cells, a large number of donor-derived myocytes were observed from the apex of the heart to the entire heart corresponding to the injured plane (Fig. 5). Fig. 5 is a stereoscopic image composed of 40 cross sections. The left ventricle is shown in the center. Scattered dots with yellow to red colors represent bone marrow-derived myocardial cells (arrow marks in Fig. 5).

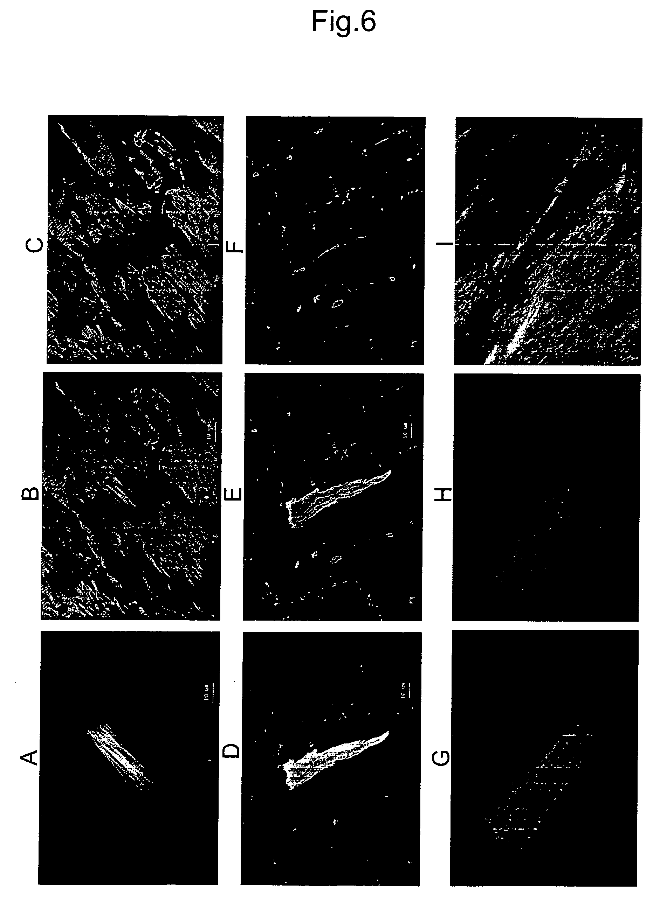

Immunological analysis was also performed by staining heart sections with connexin 43 and troponin 1c. As a result, individual antibody staining patterns were observed (Fig. 6). In Fig. 6, upper panel C shows the results of immunostaining with troponin 1c. Panel A shows the results of GFP staining alone. Panel B is a composite of panels A and C. From these results, expression of myocardial cell specific markers was clearly confirmed. Likewise, middle panel F in Fig. 6 shows the results of staining with connexin 43. Panel D shows GFP positive (i.e. derived from transplanted bone marrow cells) myocardial cells. Panel E is a composite of panels D and F. From these results, it was confirmed that myocardial cells are bone marrow-derived cells. This demonstrates that, after injury, some types of myocardial cells may be differentiated from bone marrow-derived stem cells.

In order to further perform morphological analysis, GFP positive myocardial cells were observed by Nomarski imaging (lower panels in Fig. 6). The lower panels in Fig. 6 show relationships between the contours of GFP positive cells and surrounding cells. From these panels, the contours have become clear, and it has been found that donor-derived bone marrow cells are incorporated surely and normally as myocardial cells.

From what have been described so far, it was confirmed that those myocardial cells are surely bone marrow derived cells morphologically and immunologically.

### EXAMPLE 3: Regeneration of Other Organs

### (1) Mice and Bone Marrow Cells

Breeding/administration of mice and preparation of bone marrow cells were carried out in the same manner as in Example 1. Pretreatment (irradiation) of mice and transplantation of hematopoietic cells (bone marrow transplantation) therein were also carried out in the same manner as in Example 1.

### (2) Creation of Injury Models

### (2-1) Injury Model of the Lung

Alveolar epithelial cells were injured by exposure to high concentration oxygen or by administration of LPS (endotoxin).

### (2-2) Injury Model of Kidney

Mesangial cells were injured with anti-Thy-1 antibody.

### (2-3) Injury Model of Small Intestine

Radiation enteritis was induced by irradiation.

### (2-4) Injury Model of Bone

Irradiation was performed.

### (2-5) Injury Model of Tooth and Gingiva

The gingiva was directly injured with a needle or cells were poured into the gingival.

### (2-6) Injury Model of Eye

Irradiation was performed.

### (2-7) Injury Model of Brain

Recipient mice (C57/BL6) immediately to up to 3 days after birth were anesthetized, and then donor-derived cells were transplanted in them around the ventricles.

### (3) Analysis of Differentiation

Differentiation and regeneration of the following cells were examined: bronchial epithelial cells for the lung; mesangial cells for the kidney; epithelioid cells for the small intestine; cortical bone for the bone; dental surface and gingival cells for the tooth and the gingiva; eye surface and parenchyma of cornea for the eye; and cells showing the morphologies of neuron and glia for the brain.

### (4) Results

### (4-1) Lung

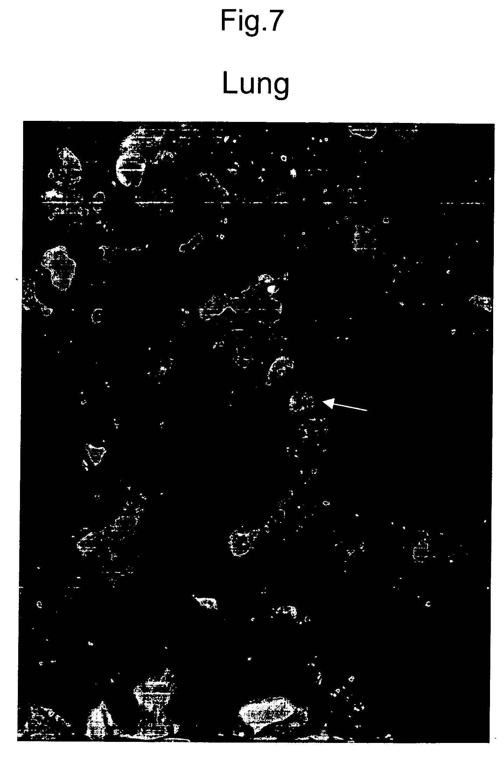

It was shown that both pulmonary and bronchial epithelial cells are regenerated in a donor's bone marrow-derived manner. Double staining with cytokeratin confirmed that GFP positive cells are epithelial cells (Fig. 7).

### (4-2) Kidney

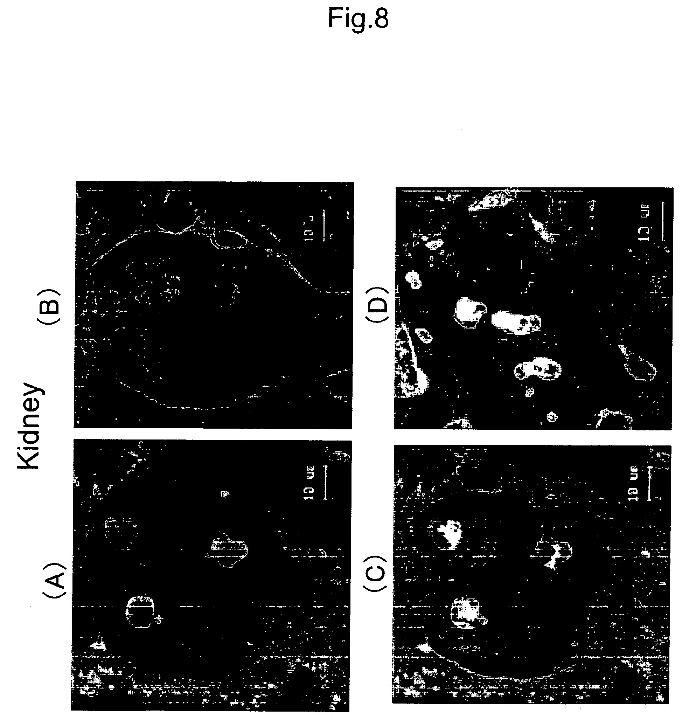

In Fig. 8, panel A is a photograph where three GFP positive cells were observed. Panel B shows the results of staining with collagen 4. Panel C is a composite of panels A and B; the three cells were identified as mesangial cells in the kidney. Panel D shows identification of donor-derived cells in the same manner as described above at a different site.

### (4-3) Small Intestine

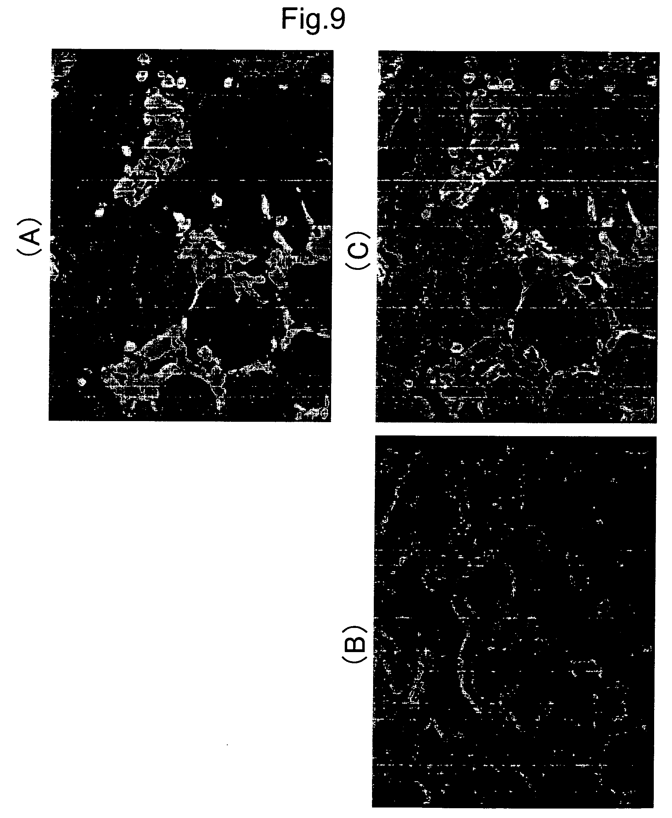

Donor-derived epithelioid cells were regenerated near the cavity of the intestinal tract (Fig. 9). In Fig. 9, panel A is a photograph where GFP positive cells were observed. Panel B shows the results of staining with pan-cytokeratin. Panel C is a composite of panels A and B.

### (4-4) Bone

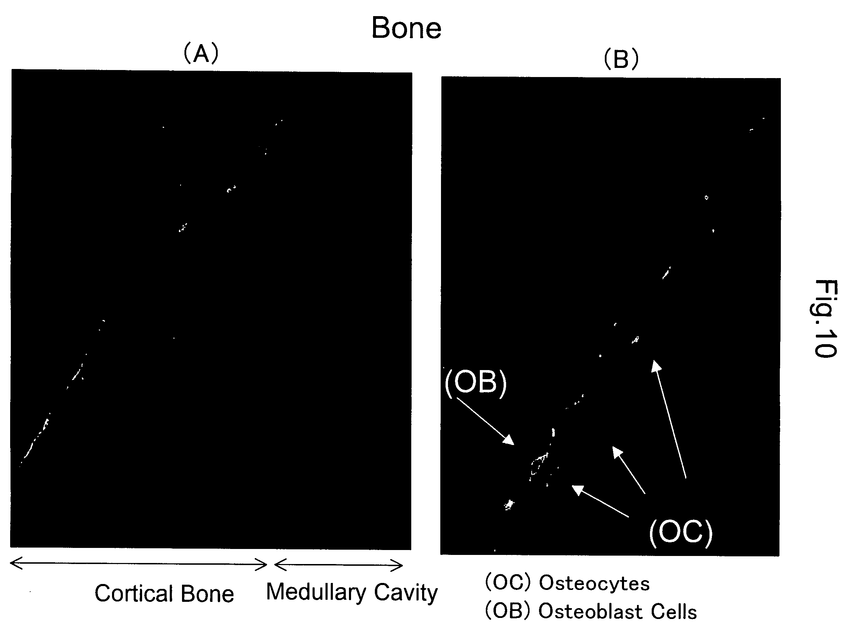

Donor-derived cells were observed in the cortical bone (Fig. 10). Fig. 10 provides photographs showing the cortical bone and the medullary cavity. Panel A is slightly enlarged and panel B is highly enlarged. "OC" represents osteocytes and "OB" osteoblast cells.

### (4-5) Tooth and Gingiva

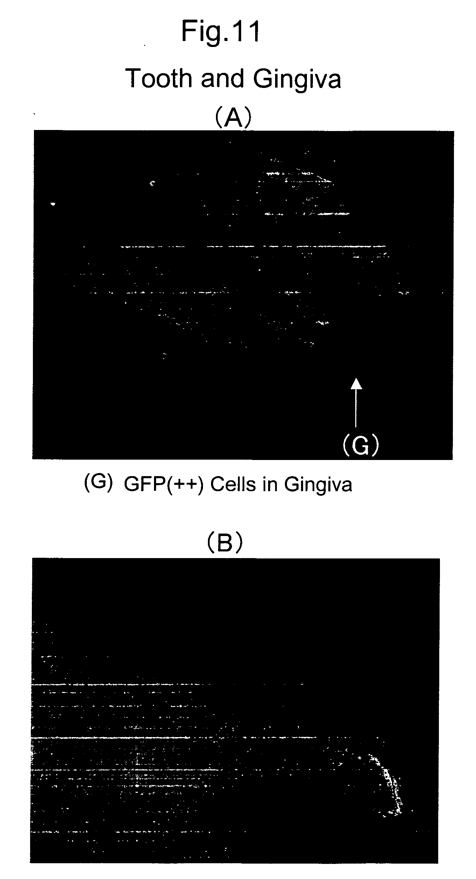

Regeneration was also recognized in the tooth and the gingiva (Fig. 11). In Fig. 11, panel A shows gingival cells and panel B shows cells near the dental surface. The mark (G) indicates GFP positive cells in the gingiva.

### (4-6) Eye

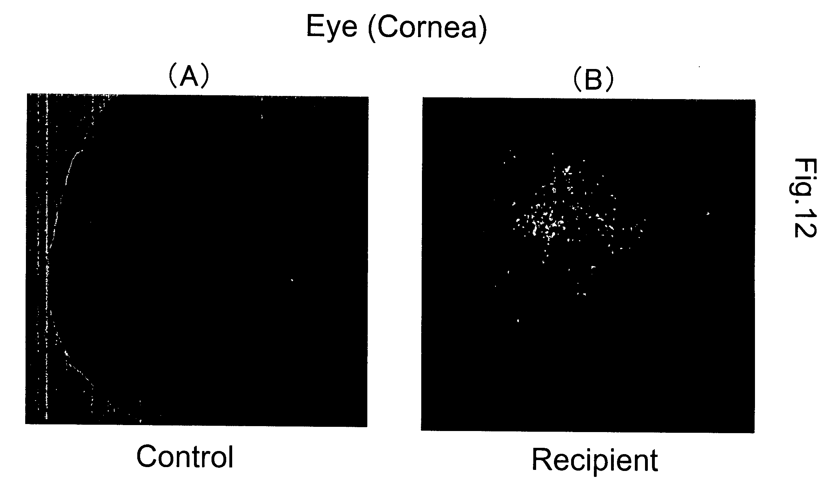

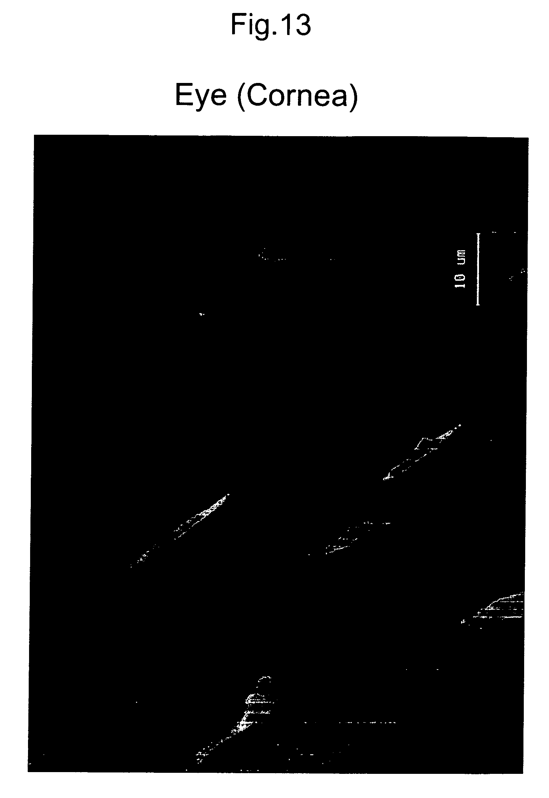

Regeneration was recognized on the surface of the eye and in corneal epithelial cells (Figs. 12 and 13). In Fig. 12, panel A shows a control where bone marrow transplantation was not performed; GFP fluorescence is not observed here. Panel B shows the eye of a recipient who received bone marrow transplantation; GFP fluorescence was recognized. Fig. 13 shows cells within the cornea; donor-derived cells (GFP fluorescence) were recognized.

### (4-7) Brain

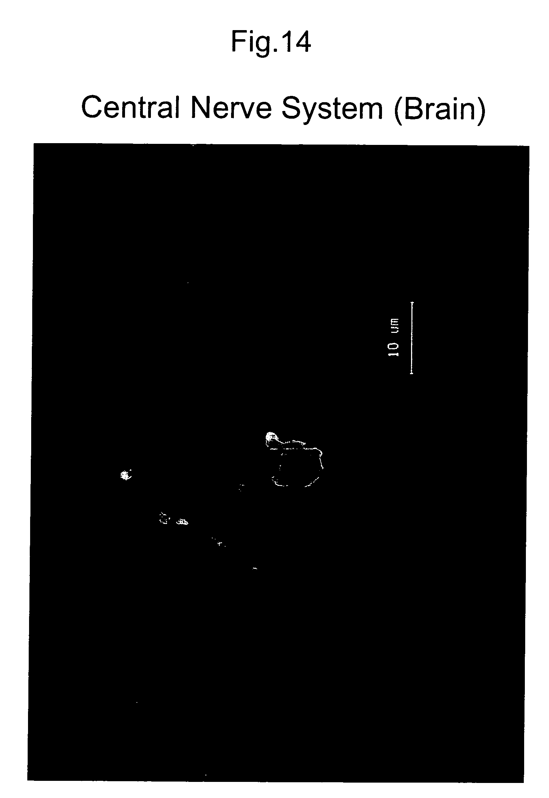

Regeneration of cells having a neurite-like construct was recognized (Fig. 14).

As a result of analysis in the brain, it was found that nerve cells derived from donor's bone marrow are differentiated.

### INDUSTRIAL APPLICABILITY

A method for regenerating tissues is provided by the present invention. According to the method of the present invention, an organ can be regenerated by performing hematopoietic cell transplantation in a mammal having impairment in the organ. Besides, by giving an injury to an organ, it is possible to construct at the injured site tissue-constituting cells derived from bone marrow or the like. Further, the thus constructed cells may be collected and used in a therapeutic method where an artificial tissue is transplanted in a patient. Still further, by creating disease animal models by injuring animals, it is also possible to replace the injured site and peripheral regions thereof with normal cells derived from bone marrow or the like. Therefore, the method of the present invention is useful in still wide-ranged regenerative medicine.

## Claims

1. A method of regenerating an organ or a part thereof, comprising performing bone marrow transplantation or hematopoietic stem cell transplantation in a mammal having an impairment in the organ or the part thereof.

2. The method according to claim 1, wherein the hematopoietic stem cell is peripheral blood hematopoietic stem cell or cord blood hematopoietic stem cell.

3. A method of treating an impairment, comprising performing bone marrow transplantation or hematopoietic stem cell transplantation in a mammal having the impairment in an organ or a part thereof and thereby regenerating the organ or the part thereof.

4. The method according to claim 3, wherein the hematopoietic stem cell is peripheral blood hematopoietic stem cell or cord blood hematopoietic stem cell.

5. A method of preparing an organ or a part thereof, comprising performing bone marrow transplantation or hematopoietic stem cell transplantation in a mammal having an impairment in the organ or the part thereof, thereby regenerating the organ or the part thereof, and recovering the resultant regenerated organ or the part thereof.

6. The method according to claim 5, wherein the hematopoietic stem cell is peripheral blood hematopoietic stem cell or cord blood hematopoietic stem cell.

7. The method according to any one of claims 1 to 6, wherein the impairment is a functional disorder of a physical or chemical injury.

8. The method according to any one of claims 1 to 7, wherein the regenerated organ or the part thereof is derived from a donor.

9. The method according to any one of claims 1 to 8, wherein the mammal is a new born mammal.

10. The method according to any one of claims 1 to 9, wherein the organ is at least one selected from the group consisting of liver, heart, brain, lung, kidney, intestine, pancreas, eye, bone and tooth.

11. An organ or a part thereof which has been regenerated by performing bone marrow transplantation or hematopoietic stem cell transplantation in a mammal having an impairment in the organ or the part thereof.

12. The organ or the part thereof according to claim 11, wherein the hematopoietic stem cell is peripheral blood hematopoietic stem cell or cord blood hematopoietic stem cell.

13. The organ or the part thereof according to claim 11 or 12, wherein the impairment is a functional disorder of a physical or chemical injury.

14. The organ or the part thereof according to any one of claims 11 to 13, wherein the regenerated organ or the part thereof is derived from a donor.

15. The organ or the part thereof according to any one of claims 11 to 14, wherein the mammal is a new born mammal.

16. The organ or the part thereof according to any one of claims 11 to 15, wherein the organ is at least one selected from the group consisting of liver, heart, brain, lung, kidney, intestine, pancreas, eye, bone and tooth.
